# EUROPÄISCHE PATENTSCHRIFT

(11) **EP 3 821 870 B1**
(45) Veröffentlichungstag und Bekanntmachung des Hinweises auf die Patenterteilung: **27.09.2023**
(21) Anmeldenummer: 20208061.0
(22) Anmeldetag: 17.11.2020
(51) Int. Cl.: A61H 9/00, A61B 5/145, A61B 5/00, A61B 5/1455

(54) **SYSTEM FÜR EINE KOMPRESSIONSTHERAPIE UMFASSEND EINEN DRUCKSENSOR UND EINEN PULSOXIMETRISCHEN SAUERSTOFFSENSOR**
SYSTEM FOR COMPRESSION THERAPY COMPRISING A PRESSURE SENSOR AND A PULSE OXIMETRIC OXYGEN SENSOR
SYSTÈME POUR UNE THÉRAPIE PAR COMPRESSION COMPRENANT UN CAPTEUR DE PRESSION ET UN CAPTEUR D'OXYGÈNE DE MESURE PAR OXYMÉTRIE DE POULS

(30) Priorität: 18.11.2019 DE 102019131055
(43) Veröffentlichungstag der Anmeldung: 19.05.2021
(73) Patentinhaber: Paul Hartmann AG, 89522 Heidenheim (DE)
(72) Erfinder: BAUER, Jochen, 89075 Ulm (DE); SMOLA, Hans, 66121 Saarbrücken (DE); ROCHE, Laurent, 89231 Neu-Ulm (DE); PEICHL, Frank, 73529 Schwäbisch Gmünd (DE); CROIZAT, Pierre, 89542 Herbrechtingen (DE); RAYER, Mathieu, 86199 Augsburg (DE); SPRENGER, Dennis, 90552 Röthenbach a. d. Pegnitz (DE); MOOS, Gunnar, 81547 München (DE); KITTLER, Jan-Philipp, 81675 München (DE)
(74) Vertreter: Paul Hartmann AG Patents & Licensing

(56) Entgegenhaltungen:
- WO-A1-2019/072531
- WO-A1-2020/049291
- US-A1- 2019 254 906

## Beschreibung

Die Erfindung betrifft ein System für eine Kompressionstherapie und ein dafür vorgesehenes Computerprogrammprodukt.

Kompressionsbinden zur Behandlung der Veneninsuffizienz sind bekannt. Zur Behandlung der Veneninsuffizienz dürfen die Kompressionsbinden weder mit einem zu niedrigen noch mit einem zu hohen Druck angelegt werden. Bei einem zu niedrigen Anlegedruck ist die Behandlung gegebenenfalls wirkungslos. Bei einem zu hohen Anlegedruck werden die behandelten Köperteile gegebenenfalls nicht mehr richtig durchblutet und geschädigt. Um den richtigen Anlegedruck einzustellen, sind Kompressionsbinden mit mechanischen Druckindikatoren sowie mit elektronischen Drucksensoren bekannt.

Allerdings ist die Kontrolle des Anlegedrucks nicht in allen Fällen ausreichend, um eine komplikationslose Behandlung der Veneninsuffizienz zu gewährleisten. Zum Beispiel kann die alleinige Kontrolle des Anlegedrucks bei Patienten, welche gleichzeitig an einer Veneninsuffizienz und einer arteriellen Verschlusskrankheit (AVK) leiden, nicht ausreichend sein. Bei dieser Risikogruppe kann ein normalerweise unproblematischer Anlegedruck zu einer Minderperfusion der betroffenen Extremität und infolge dessen zu einem Untergang von Gewebe führen. Deshalb wird bei diesen Patienten aus Sicherheitsgründen oftmals keine Kompressionstherapie angewandt und deren Veneninsuffizienz unbehandelt gelassen, obwohl eine Kompressionstherapie unter Umständen möglich wäre und zu einer verbesserten Blutversorgung durch Abnahme der venösen Hypertension und somit zu einer Erhöhung der Druckdifferenz zwischen dem Blutdruck im arteriellen und venösen Kompartiment führen könnte.

Die US 2019/0254906 A1 offenbart ein System zur Bereitstellung einer Kompressionsbehandlung.

Das System umfasst eine tragbare Kompressionsvorrichtung mit
- einem motorbetriebenen Kompressionsmechanismus,
- einem oder mehreren Sensoren zum Messen von physiologischen Daten und/oder Leistungsdaten der Vorrichtung,
- einer Steuereinheit zum Steuern der Vorrichtung gemäß einem Satz von Parametern und
- einem drahtlosen Kommunikationsmodul in Kommunikation mit der Steuereinheit und dem einen oder mehreren Sensoren.

Weiterhin umfasst das System ein Fernbedienungsgerät wie beispielsweise ein Smartphone, welches drahtlos mit dem Kommunikationsmodul der Kompressionsvorrichtung kommuniziert und die gemessenen physiologischen Daten und/oder die Leistungsdaten der Vorrichtung empfängt und den Parametersatz zur Steuerung der Vorrichtung moduliert.

WO 2020/049291 A1 offenbart ein System umfassend eine optische Faseranordnung, die so konfiguriert ist, dass sie einen oder mehrere physiologische Parameter eines Individuums misst. Weiterhin umfasst das System einen Drucksensor, der so konfiguriert ist, dass er einen Kontaktdruck der optischen Faseranordnung auf das Individuum misst. Das System kann in eine Kompressionsbandage integriert sein.

Der vorliegenden Erfindung lag die Aufgabe zugrunde, die Sicherheit bei einer Kompressionstherapie zu verbessern. Diese Aufgabe wird mit einem System nach Anspruch 1 und einem Computerprogrammprodukt nach Anspruch 17 gelöst.

Erfindungsgemäß umfasst das System für eine Kompressionstherapie ein Kompressionsmittel, einen Drucksensor und einen Computer. Das System ist dadurch gekennzeichnet, dass es einen pulsoximetrischen Sauerstoffsensor umfasst, wobei Messwerte des Drucksensors und des Sauerstoffsensors an den Computer übertragen werden können. Weiterhin ist das System dadurch gekennzeichnet, dass der Computer Mittel zur Ausführung eines Verfahrens zur Überprüfung der Kompressionstherapie umfasst, wobei das Verfahren die folgenden Schritte umfasst:
i. Empfangen und Prüfen eines Messwertes des Drucksensors während der Kompressionstherapie,
   wobei geprüft wird, ob der Messwert des Drucksensors außerhalb eines vorgegebenen Druckbereiches liegt, und
   wobei vorzugsweise ein Alarm ausgegeben wird, wenn die Prüfung ergibt, dass der Messwert des Drucksensors außerhalb des vorgegebenen Druckbereiches liegt.
ii. Empfangen und Prüfen eines Messwertes des Sauerstoffsensors während der Kompressionstherapie,
   wobei geprüft wird, ob der Messwert des Sauerstoffsensors eine vorgegebene Untergrenze für eine Sauerstoffsättigung unterschreitet, und
   wobei vorzugsweise ein Alarm ausgegeben wird, wenn die Prüfung ergibt, dass der Messwert des Sauerstoffsensors die vorgegebene Untergrenze für die Sauerstoffsättigung unterschreitet.

Bei den zuvor genannten Mitteln des Computers handelt es sich insbesondere um einen Prozessor und ein Computerprogramm. Letzteres ist Gegenstand des Anspruchs 17 und wird im Folgenden noch genauer beschrieben.

Gegebenenfalls kann eine Weiterverarbeitung der Messwerte des Drucksensors und insbesondere des Sauerstoffsensors erforderlich sein, damit der Abgleich mit den im vorliegenden Dokument genannten Referenzbereichen und Grenzwerten erfolgen kann. Eine solche Weiterverarbeitung der Messwerte kann von dem Computer im Rahmen der entsprechenden Prüfungen durchgeführt werden. Zudem kann es für die Berechnung einer arteriellen Sauerstoffsättigung sowie eines Pulsschlages notwendig sein, dass mehrere Messwerte von dem Sauerstoffsensor in einer zeitlichen Abfolge erfasst und von dem Computer weiterverarbeitet werden. Daher kann der Messwert aus dem obigen Verfahrensschritt ii. auch mehrere Messwerte umfassen, welche in einer zeitlichen Abfolge von dem Sauerstoffsensor erfasst und von dem Computer zu einer gemessenen Sauerstoffsättigung (oder auch eines gemessenen Pulsschlages) weiterverarbeitet werden.

Das erfindungsgemäße Kompressionstherapiesystem überwacht also zum einen den Druck, der auf das behandelte Körperteil des Patienten einwirkt. Zusätzlich überwacht das erfindungsgemäße Kompressionstherapiesystem auch die Sauerstoffsättigung des arteriellen Blutes in dem behandelten Körperteil des Patienten. Mit der ersten Maßnahme - der Drucküberwachung - kann insbesondere ein akutes Versagen der Kompressionstherapie bei einem zu niedrigen Druck oder eine akute Gefahrensituation infolge der Kompressionstherapie bei einem zu hohen Druck erfasst werden. Die zweite Maßnahme - die Überwachung der Sauerstoffsättigung - dient insbesondere dazu, eine Mangeldurchblutung des behandelten Körperteils infolge einer für den Patienten zu starken Kompression zu erfassen. Eine solche Mangeldurchblutung kann bei den zuvor genannten Risikopatienten bereits dann auftreten, wenn der Druck den oben erwähnten Druckbereich noch gar nicht überschritten hat. Durch die gleichzeitige Überwachung von Druck und Sauerstoffsättigung wird jedenfalls die Sicherheit des Kompressionstherapiesystems erhöht, wodurch die Kompressionstherapie einem größeren Patientenkreis zugänglich gemacht werden kann. Weitere Vorteile der Erfindung ergeben sich aus den nachfolgend beschriebenen bevorzugten Ausführungsformen.

Das Kompressionsmittel kann eine Kompressionsbinde, ein Kompressionsschlauch oder ein Kompressionsstrumpf sein. Die Kompressionsbinde hat den Vorteil, dass sie an verschiedene Körperteile angelegt werden kann. Der Kompressionsschlauch und der Kompressionsstrumpf können leicht und schnell angelegt werden. Kompressionsbinden sind im Handel erhältlich, zum Beispiel von der Patentanmelderin Paul Hartmann AG (Heidenheim, Deutschland) unter dem Markennamen Pütter^{®}. Es können auch mehrere der zuvor genannten Kompressionsmittel miteinander kombiniert werden. Zum Beispiel kann eine Kompressionsbinde mit einem Kompressionsstrumpf kombiniert eingesetzt werden.

Bei dem Drucksensor kann es sich um einen Dehnungsmessstreifen, einen induktiven Drucksensor oder einen piezoelektrischen Drucksensor handeln. Bevorzugt wird aus dieser Gruppe der piezoelektrische Drucksensor. Der Dehnungsmessstreifen ist vorteilhafterweise in das Kompressionsmittel, insbesondere in die Kompressionsbinde, integriert oder zumindest mit diesem verbunden.

Typischerweise umfasst der Sauerstoffsensor eine Lichtquelle für rotes Licht, eine Lichtquelle für infrarotes Licht und einen Photodetektor. Die Lichtquellen sind vorzugsweise Leuchtdioden (light-emitting diode, LED). Der Photodetektor ist vorzugsweise eine Photodiode. Ein solcher Sauerstoffsensor wird unter der Bezeichnung BIOFY^{®} SFH7060 von Osram Opto Semiconductors (Regensburg, Deutschland) vertrieben. Die Lichtquellen können dafür verwendet werden, um Licht der Wellenlängen 660 nm (rotes Licht) und 940 nm (infrarotes Licht) zu erzeugen. Der Photodetektor kann das durch das Gewebe hindurchtretende beziehungsweise von dem Gewebe reflektierte Licht erfassen, woraufhin von dem Computer über im Stand der Technik an sich bekannte Verfahren die Sauerstoffsättigung des arteriellen Blutes berechnet werden kann. Entsprechend kann der anspruchsgemäße Messwert des Sauerstoffsensors Messwerte des Photodetektors im Hinblick auf das transmittierte beziehungsweise reflektierte rote Licht sowie Messwerte des Photodetektors im Hinblick auf das transmittierte beziehungsweise reflektierte infrarote Licht umfassen.

Weiterhin handelt es sich bei dem Sauerstoffsensor vorzugsweise um einen reflexions-pulsoximetrischen Sauerstoffsensor. Das bedeutet, dass der pulsoximetrische Sauerstoffsensor vorzugsweise das Prinzip der Reflexionsoximetrie nutzt. Damit wird die Anordnung des Sauerstoffsensors am Körper des Patienten deutlich vereinfacht. Die zuvor genannten Bestandteile des Sauerstoffsensors (Lichtquellen und Detektor) müssen dann nämlich nicht wie bei der alternativen Transmissionsoximetrie an sich gegenüberliegenden Körperstellen angeordnet werden, sondern können an derselben Körperstelle platziert werden.

Gemäß einer bevorzugten Ausführungsform der Erfindung ist zudem vorgesehen, dass das System mehrere Drucksensoren und/oder mehrere pulsoximetrische Sauerstoffsensoren umfasst. Dadurch können an verschiedenen Stellen unter dem Kompressionsmittel Druck und Sauerstoffsättigung bestimmt werden. Die Genauigkeit des Systems wird erhöht. Wenn das Kompressionstherapiesystem wie in dieser bevorzugten Ausführungsform vorgesehen mehrere Drucksensoren und/oder mehrere pulsoximetrische Sauerstoffsensoren umfasst, kann jeder der zusätzlichen Sensoren auch gemäß der zuvor beschriebenen Ausführungsformen ausgebildet sein. Weiterhin kann dann jeder der zusätzlichen Sensoren insofern dies nicht bereits explizit vorgesehen ist auch ein Teil der nachfolgend beschriebenen Ausführungsformen sein. Wenn also in einer nachfolgend genannten Ausführungsform des Kompressionstherapiesystems von einem Drucksensor oder einem Sauerstoffsensor gesprochen wird, können bei Kombination dieser nachfolgenden Ausführungsform mit der vorliegenden Ausführungsform auch mehrere Drucksensoren oder mehrere Sauerstoffsensoren gemeint sein.

Bei Vorhandensein von mehreren Drucksensoren kann in Schritt i. des Verfahrens zur Überprüfung der Kompressionstherapie von jedem Drucksensor jeweils ein Messwert empfangen werden, wobei die Messwerte der Drucksensoren zusammengefasst werden, um einen einzelnen, zusammengefassten Drucksensor-Messwert zu bilden. Der zusammengefasste Drucksensor-Messwert wird dann dahingehend geprüft, ob er außerhalb des vorgegebenen Druckbereiches liegt. Vorzugsweise wird der Alarm ausgegeben, wenn die Prüfung ergibt, dass der zusammengefasste Drucksensor-Messwert außerhalb des vorgegebenen Druckbereiches liegt. Bevorzugt ist der zusammengefasste Drucksensor-Messwert ein Mittelwert.

Schritt i. des Verfahrens zur Überprüfung der Kompressionstherapie könnte bei Vorhandensein von mehreren Drucksensoren demnach lauten:
Empfangen und Prüfen jeweils eines Messwertes von jedem (der mehreren) Drucksensor(en) während der Kompressionstherapie,
wobei die Messwerte der Drucksensoren zusammengefasst werden, um einen einzelnen, zusammengefassten Drucksensor-Messwert zu bilden,
wobei geprüft wird, ob der zusammengefasste Drucksensor-Messwert außerhalb eines vorgegebenen Druckbereiches liegt, und
wobei vorzugsweise ein Alarm ausgegeben wird, wenn die Prüfung ergibt, dass der zusammengefasste Drucksensor-Messwert außerhalb des vorgegebenen Druckbereiches liegt.

Bei Vorhandensein von mehreren Sauerstoffsensoren kann in Schritt ii. des Verfahrens zur Überprüfung der Kompressionstherapie von jedem Sauerstoffsensor jeweils ein Messwert empfangen werden, wobei die Messwerte der Sauerstoffsensoren zusammengefasst werden, um einen einzelnen, zusammengefassten Sauerstoffsensor-Messwert zu bilden. Der zusammengefasste Sauerstoffsensor-Messwert wird dann dahingehend geprüft, ob er die vorgegebene Untergrenze für die Sauerstoffsättigung unterschreitet. Vorzugsweise wird der Alarm ausgegeben, wenn die Prüfung ergibt, dass der zusammengefasste Sauerstoffsensor-Messwert die vorgegebene Untergrenze unterschreitet. Bevorzugt ist der zusammengefasste Sauerstoffsensor-Messwert ein Mittelwert.

Schritt ii. des Verfahrens zur Überprüfung der Kompressionstherapie könnte bei Vorhandensein von mehreren Sauerstoffsensoren demnach lauten:
Empfangen und Prüfen jeweils eines Messwertes von jedem (der mehreren) Sauerstoffsensor(en) während der Kompressionstherapie,
wobei die Messwerte der Sauerstoffsensoren zusammengefasst werden, um einen einzelnen, zusammengefassten Sauerstoffsensor-Messwert zu bilden,
wobei geprüft wird, ob der zusammengefasste Sauerstoffsensor-Messwert eine vorgegebene Untergrenze für eine Sauerstoffsättigung unterschreitet, und
wobei vorzugsweise ein Alarm ausgegeben wird, wenn die Prüfung ergibt, dass der zusammengefasste Sauerstoffsensor-Messwert die vorgegebene Untergrenze für die Sauerstoffsättigung unterschreitet.

Alternativ kann bei Vorhandensein von mehreren Druck- und Sauerstoffsensoren von jedem Druck- und Sauerstoffsensor jeweils ein Messwert empfangen und separat geprüft werden. Damit können die Messstellen einzeln im Hinblick auf Druck und Sauerstoffsättigung überwacht werden, was vorliegend als besonders vorteilhaft angesehen wird.

Vorteilhaft ist es ebenso, wenn das System eine Sensoreinheit mit einem Substrat umfasst, wobei der Drucksensor und der Sauerstoffsensor auf dem Substrat angeordnet sind. Der Drucksensor und der Sauerstoffsensor bilden dann also eine gemeinsame Sensoreinheit. Dadurch können sich der Drucksensor und der Sauerstoffsensor in räumlicher Nähe zueinander befinden, so dass an derselben Körperstelle sowohl der Druck als auch die Sauerstoffsättigung gemessen werden kann. Zudem wird das System dadurch hinsichtlich seiner Struktur sowie Handhabbarkeit vereinfacht. Normalerweise befinden sich auf einer solchen Sensoreinheit lediglich ein einziger Drucksensor sowie ein einziger Sauerstoffsensor. Bezugnehmend auf die vorangegangene bevorzugte Ausführungsform der Erfindung können auch mehrere solcher Sensoreinheiten vorhanden sein, wenn das System mehrere Druck- und Sauerstoffsensoren umfasst.

Bei dem Substrat der Sensoreinheit kann es sich um eine Leiterplatte, insbesondere um eine flexible Leiterplatte, handeln. Die Befestigung der Sensoreinheit am Körper des Patienten beziehungsweise an dem Kompressionsmittel kann erleichtert werden, indem die Leiterplatte flexibel ausgebildet ist. Die flexible Leiterplatte verbessert insbesondere auch den Tragekomfort des Kompressionstherapiesystems. Bevorzugt handelt es sich bei der flexiblen Leiterplatte um einen Polymerfilm, auf dem elektronische Komponenten aufgebracht, insbesondere aufgedruckt, sind.

Darüber hinaus kann die Sensoreinheit eine Datenerfassungseinheit umfassen. Die Datenerfassungseinheit ist dafür ausgebildet die Messwerte von dem Drucksensor und dem Sauerstoffsensor zu erfassen und an den Computer zu übertragen. Ebenso wie die Sensoren kann die Datenerfassungseinheit auf dem Substrat der Sensoreinheit angeordnet sein. Sie kann aber auch mit einem Konnektor, beispielsweise einem Kabel, mit dem Substrat der Sensoreinheit verbunden sein. Es ist auch möglich, dass die Datenerfassungseinheit den Computer bei der Prüfung der Messwerte des Drucksensors und des Sauerstoffsensors unterstützt. Zum Beispiel könnte die Datenerfassungseinheit die zuvor beschriebene Weiterverarbeitung der Messwerte des Drucksensors und des Sauerstoffsensors durchführen. Die Datenerfassungseinheit kann einen Microcontroller, einen Vorverstärker und einen Analog-Digital-Wandler umfassen. Weitere mögliche elektronische Komponenten der Datenerfassungseinheit werden nachfolgend genannt. Somit kann die Datenerfassungseinheit für sich gesehen gleichfalls einen Computer darstellen.

Gemäß einer besonders bevorzugten Ausführungsform der Erfindung ist die Sensoreinheit adhäsiv ausgebildet und wird losgelöst von dem Kompressionsmittel bereitgestellt. Dafür umfasst die Sensoreinheit vorzugsweise eine adhäsive Rückschicht, wobei die Sensoreinheit dann in der Art eines Pflasters ausgestaltet sein kann. Diese Ausführungsform hat die besonderen Vorteile, dass sich die Sensoreinheit leicht und ohne zusätzliche Hilfsmittel (wie etwa einem Heftpflaster) am Körper des Patienten anbringen lässt und der Patient oder das Pflegepersonal frei wählen kann, an welcher Stelle die Sensoreinheit zur Erhebung der Messwerte platziert werden soll. Andererseits kann es auch vorgesehen sein, dass die Sensoreinheit mit dem Kompressionsmittel herstellerseitig verbunden ist. Dadurch kann das Anlegen des Systems vereinfacht werden und schneller erfolgen. Diese Variante ist in Verbindung mit der zuvor genannten flexiblen Leiterplatte für die Sensoreinheit auch insbesondere dann vorteilhaft, wenn der Drucksensor als Dehnungsmessstreifen ausgebildet ist.

Vorzugsweise handelt es sich bei dem Computer um ein mobiles Gerät. Als mobiles Gerät kommt für die Erfindung insbesondere ein Smartphone oder ein Tablet in Betracht. Dadurch kann die Anzahl der elektronischen Komponenten minimiert werden, weil auf ein bereits vorhandenes, leistungsstarkes Gerät des Patienten oder des Pflegepersonals für die Datenauswertung und Datenanzeige zurückgegriffen werden kann. Zudem kann damit die Handhabung und der Tragekomfort des Kompressionstherapiesystems verbessert werden.

In einer einfachen Ausgestaltung der Erfindung werden die Messwerte des Drucksensors und des Sauerstoffsensors an den Computer kabelgebunden übertragen. Viel vorteilhafter ist es aber, wenn die Übertragung der Messwerte des Drucksensors und des Sauerstoffsensors an den Computer kabellos erfolgt, wobei die Übertragung vorzugsweise über eine Nahfeldkommunikation (near field communication, NFC), Bluetooth oder ein drahtloses lokales Netzwerk (wireless local area network, WLAN) stattfindet. Dadurch können die Messwerte an einen beliebigen Ort übertragen und die Handhabung sowie der Tragekomfort des Systems verbessert werden. Für den kabellosen Datenaustausch kann das Kompressionstherapiesystem einen oder mehrere NFC-Sendeempfänger, Bluetooth-Sendeempfänger und/oder WLAN-Sendeempfänger umfassen. Ein NFC-Sendeempfänger, ein Bluetooth-Sendeempfänger und/oder ein WLAN-Sendeempfänger kann insbesondere in der Datenerfassungseinheit und dem Computer enthalten sein.

Um die Datensicherheit des Kompressionstherapiesystems zu verbessern, wird außerdem vorgeschlagen, dass die Übertragung der Messwerte des Drucksensors und des Sauerstoffsensors an den Computer verschlüsselt erfolgt. Die Verschlüsselung kann zum Beispiel von der Datenerfassungseinheit durchgeführt werden. Die Entschlüsselung der Messwerte kann von dem Computer durchgeführt werden.

Ein weiterer Vorteil ergibt sich, wenn der Drucksensor und der Sauerstoffsensor über eine eigene Energiequelle verfügen, wobei die Energiequelle vorzugsweise eine Batterie ist. Das heißt, dass das Kompressionstherapiesystem vorteilhafterweise weiterhin mindestens eine Energiequelle, vorzugsweise mindestens eine Batterie, für den Drucksensor und den Sauerstoffsensor umfasst. Dabei kann sowohl der Drucksensor als auch der Sauerstoffsensor von einer einzigen Energiequelle versorgt werden. Der Drucksensor und der Sauerstoffsensor können jedoch auch jeweils über eine eigene Energiequelle verfügen. Durch die Energiequelle kann eine permanente Aktivierung der Sensoren, also ein kontinuierliches Monitoring der Druck- und Sauerstoffverhältnisse, ermöglicht werden. Die Energiequelle kann von der Sensoreinheit umfasst und zum Beispiel auf deren Substrat angeordnet sein.

Denkbar ist allerdings auch, dass der Drucksensor und der Sauerstoffsensor über keine eigene Energiequelle verfügen. Das Kompressionstherapiesystem, insbesondere die Sensoreinheit, umfasst dann keine eigene Energiequelle für den Drucksensor und den Sauerstoffsensor. Dadurch kann das System vereinfacht und dessen Tragekomfort erhöht werden. Die Energieversorgung der Sensoren in dieser speziellen Ausgestaltung der Erfindung kann zum Beispiel mittels NFC-Funksignalen des verwendeten Smartphones erfolgen.

Zudem können vorteilhafte Ausführungsformen der Erfindung im Hinblick auf die Arbeitsweise des Computers angegeben werden. So wird zum Beispiel bevorzugt, dass das Empfangen und Prüfen der Messwerte des Drucksensors und des Sauerstoffsensors unmittelbar nachdem die Messwerte von den Sensoren erfasst wurden erfolgt. Dadurch kann der Benutzer des Systems über den aktuellen Zustand der Kompressionstherapie informiert werden. Ebenso wird bevorzugt, dass das Empfangen und Prüfen des Messwertes des Drucksensors und das Empfangen und Prüfen des Messwertes des Sauerstoffsensors gleichzeitig erfolgen. Dadurch kann der Benutzer des Systems umfassend über den Zustand der Kompressionstherapie informiert werden.

Weiterhin kann das Empfangen und Prüfen der Messwerte des Drucksensors und des Sauerstoffsensors automatisch und kontinuierlich nach Beginn der Kompressionstherapie erfolgen oder zumindest automatisch in regelmäßigen Abständen nach Beginn der Kompressionstherapie erfolgen. Die Überprüfung der Messwerte kann dann nicht vergessen werden, was die Sicherheit des Systems erhöht. Alternativ dazu kann das Empfangen und Prüfen der Messwerte des Drucksensors und des Sauerstoffsensors ausschließlich auf Befehl eines Benutzers hin erfolgen. Dadurch kann das System vereinfacht werden, weil dann zum Beispiel auf eine Energiequelle für die Sensoren verzichtet werden kann.

Der von dem Computer zur Überprüfung des Druck-Messwertes herangezogene Druckbereich hat eine Untergrenze und eine Obergrenze. Die Untergrenze des Druckbereiches kann einen Wert von 1 mmHg bis 20 mmHg, bevorzugt 10 mmHg bis 20 mmHg und besonders bevorzugt 15 mmHg bis 20 mmHg aufweisen. Die Obergrenze des Druckbereiches kann einen Wert von 40 mmHg bis 200 mmHg, bevorzugt 40 mmHg bis 120 mmHg, mehr bevorzugt 40 mmHg bis 100 mmHg, noch mehr bevorzugt 40 mmHg bis 80 mmHg und besonders bevorzugt 40 mmHg bis 60 mmHg aufweisen. Insbesondere weist die Untergrenze einen Wert von 20 mmHg und die Obergrenze einen Wert von 40 mmHg auf, so dass sich ein Druckbereich von 20 mmHg bis 40 mmHg ergibt. Ein Alarm würde dann ausgegeben werden, wenn der gemessene Druck außerhalb des Druckbereiches von 20 mmHg bis 40 mmHg liegt. Dabei würde sowohl ein Messdruck unter 20 mmHg als auch ein Messdruck über 40 mmHg als außerhalb des Druckbereiches liegend angesehen werden. Bei einem gemessenen Druck innerhalb des zuvor genannten Druckbereiches, also bei einem Messdruck von 20 mmHg bis 40 mmHg, würde hingegen keine Alarmmeldung erfolgen.

Die mittels Pulsoximetrie bestimmte Sauerstoffsättigung ist normalerweise ein prozentualer Wert. Dieser gibt an, wieviel Prozent des roten Blutfarbstoffes Hämoglobin mit Sauerstoff beladen ist. Als unbedenklich und im Normbereich liegend können Werte über 95 % angesehen werden. Entsprechend kann die Untergrenze für die Sauerstoffsättigung aus einem Bereich von 95 % bis 70 %, vorzugsweise 90 % bis 70 %, ausgewählt sein. So kann die Untergrenze für die Sauerstoffsättigung beispielsweise 95 %, 90 %, 85 %, 80 %, 75 % oder 70 % betragen. Insbesondere beträgt die Untergrenze für die Sauerstoffsättigung 90 % oder 85 %.

In einer besonders bevorzugten Ausführungsform der Erfindung wird für die Untergrenze der Sauerstoffsättigung ein patientenbezogener Wert herangezogen. Dabei ist die Untergrenze für die Sauerstoffsättigung eine vor Beginn oder zu Beginn der Kompressionstherapie von dem Sauerstoffsensor gemessene Sauerstoffsättigung des zu behandelnden beziehungsweise behandelten Körperteils. Dadurch kann die Konstitution des Patienten bei der Überwachung der Kompressionstherapie berücksichtigt werden.

Die Pulsoximetrie erfasst neben der Sauerstoffsättigung des arteriellen Blutes auch den Pulsschlag. Entsprechend kann der Computer vorteilhafterweise weiterhin Mittel zur Ausführung eines Verfahrens zur Überprüfung eines Pulsschlages umfassen. In diesem Verfahren empfängt der Computer während der Kompressionstherapie einen Messwert des Sauerstoffsensors und prüft diesen dahingehend, ob er außerhalb eines vorgegebenen Pulsbereiches liegt. Vorzugsweise wird ein Alarm ausgegeben, wenn die Prüfung ergibt, dass der Messwert des Sauerstoffsensors außerhalb des vorgegebenen Pulsbereiches liegt. Bei dem hierbei verwendeten Messwert kann es sich um denselben Messwert handeln, welcher zur Überprüfung der Kompressionstherapie herangezogen wird. Der Pulsschlag kann Auskunft über den Kreislaufzustand des behandelten Patienten geben und für den Anwender des Kompressionstherapiesystems gleichfalls von Interesse sein. Für die Erfassung des Pulsschlages kann es vorteilhaft sein, wenn der Sauerstoffsensor auch eine Lichtquelle für grünes Licht, insbesondere eine grüne LED, umfasst.

Falls mehrere Sauerstoffsensoren vorhanden sind, kann in dem Verfahren zur Überprüfung des Pulsschlages von jedem Sauerstoffsensor jeweils ein Messwert empfangen werden, wobei die Messwerte der Sauerstoffsensoren zusammengefasst werden, um einen einzelnen, zusammengefassten Sauerstoffsensor-Messwert zu bilden. Der zusammengefasste Sauerstoffsensor-Messwert wird dann dahingehend geprüft, ob er außerhalb des vorgegebenen Pulsbereiches liegt. Vorzugsweise wird der Alarm ausgegeben, wenn die Prüfung ergibt, dass der zusammengefasste Sauerstoffsensor-Messwert außerhalb des vorgegebenen Pulsbereiches liegt. Bevorzugt ist der zusammengefasste Sauerstoffsensor-Messwert ein Mittelwert.

Das Verfahren zur Überprüfung des Pulsschlages könnte bei Vorhandensein von mehreren Sauerstoffsensoren demnach lauten:
Empfangen und Prüfen jeweils eines Messwertes von jedem (der mehreren) Sauerstoffsensor(en) während der Kompressionstherapie,
wobei die Messwerte der Sauerstoffsensoren zusammengefasst werden, um einen einzelnen, zusammengefassten Sauerstoffsensor-Messwert zu bilden,
wobei geprüft wird, ob der zusammengefasste Sauerstoffsensor-Messwert außerhalb eines vorgegebenen Pulsbereiches liegt, und
wobei vorzugsweise ein Alarm ausgegeben wird, wenn die Prüfung ergibt, dass der zusammengefasste Sauerstoffsensor-Messwert außerhalb des vorgegebenen Pulsbereiches liegt.

Alternativ dazu könnte von jedem Sauerstoffsensor jeweils ein Messwert empfangen und im Hinblick auf den vorgegebenen Pulsbereich separat geprüft werden.

Wie der Druckbereich hat auch der Pulsbereich eine Untergrenze und eine Obergrenze. Die Untergrenze des Pulsbereiches kann einen Wert von 40 Schläge/min bis 60 Schläge/min, insbesondere 50 Schläge/min, aufweisen. Die Obergrenze des Pulsbereiches kann einen Wert von 100 Schläge/min bis 200 Schläge/min, insbesondere 150 Schläge/min, aufweisen. Bevorzugt wird also ein Pulsbereich von 50 Schläge/min bis 150 Schläge/min.

Wie bereits erwähnt sind Alarme für den Fall vorgesehen, dass die Messwerte den vorgegebenen Referenzbereichen und Grenzwerten nicht entsprechen. Dabei kann es sinnvoll sein, wenn die Ausgabe der Alarme solange verzögert wird, bis das Ergebnis der Prüfungen einmal oder mehrmals bestätigt wurde. Falsche Alarmmeldungen infolge von nur kurzzeitigen beziehungsweise vorübergehenden Veränderungen von Druck, Sauerstoffsättigung oder Pulsschlag können so vermieden werden. Die hiermit vorgeschlagene Alarmverzögerung knüpft insbesondere an die zuvor genannte Ausführungsform an, in der das Empfangen und Prüfen der Messwerte des Drucksensors und des Sauerstoffsensors automatisch und kontinuierlich oder zumindest automatisch in regelmäßigen Abständen nach Beginn der Kompressionstherapie erfolgt. Typischerweise werden die Alarme visuell und/oder akustisch ausgegeben.

Üblicherweise umfasst das System weiterhin ein Eingabemittel und ein Ausgabemittel. Das Eingabemittel und das Ausgabemittel sind vorzugsweise Bestandteile des Computers. Das Eingabemittel kann dazu dienen, Befehle oder Vorgaben des Benutzers entgegenzunehmen. Das Ausgabemittel kann dazu dienen, die gemessenen Druck-, Sauerstoffsättigungs- und Pulsschlag-Werte anzuzeigen und die Alarme auszugeben. Als sowohl Eingabemittel als auch Ausgabemittel kann ein Berührungsbildschirm (touch screen) von beispielsweise einem Smartphone oder Tablet fungieren. Das Ausgabemittel kann auch ein Lautsprecher sein (akustische Alarme). Jedes Smartphone oder Tablet verfügt heutzutage über einen Berührungsbildschirm und einen Lautsprecher.

Normalerweise umfasst das System auch einen Datenträger. Auch der Datenträger ist vorzugsweise Bestandteil des Computers. Der Datenträger kann dazu dienen, Computerprogramme und die empfangenen Messwerte zu speichern. Dadurch kann der Benutzer den gesamten Verlauf der Kompressionstherapie mit allen empfangenen Messwerten nachvollziehen. Bei dem Datenträger kann es sich um eine Festplatte, ein Halbleiterlaufwerk (solid-state-drive, SSD) oder eine SD-Karte (secure digital memory card) handeln. Das Kompressionstherapiesystem kann auch mehr als einen Datenträger besitzen. Insbesondere die zuvor genannte Datenerfassungseinheit kann auch einen Datenträger umfassen. Denkbar ist zudem, dass die Sensormesswerte von dem Kompressionstherapiesystem in einem onlinebasierten Datenspeicherdienst (Cloud) oder auf einem lokalen Netzlaufwerk gespeichert werden.

Offenbart wird ebenso ein computerimplementiertes Verfahren zur Überprüfung einer Kompressionstherapie. Dieses Verfahren umfasst die folgenden Schritte:
i. Erzeugen eines Messwertes mit einem Drucksensor, um einen von einem Kompressionsmittel auf eine Körperstelle ausgeübten Druck während der Kompressionstherapie zu bestimmen,
ii. Erzeugen eines Messwertes mit einem pulsoximetrischen Sauerstoffsensor, um eine Sauerstoffsättigung der mit dem Kompressionsmittel behandelten Körperstelle während der Kompressionstherapie zu bestimmen,
iii. Übertragen der Messwerte des Drucksensors und des Sauerstoffsensors an einen Computer,
iv. Empfangen und Prüfen des Messwertes des Drucksensors während der Kompressionstherapie durch den Computer,
   wobei geprüft wird, ob der Messwert des Drucksensors außerhalb eines vorgegebenen Druckbereiches liegt, und
   wobei vorzugsweise ein Alarm ausgegeben wird, wenn die Prüfung ergibt, dass der Messwert des Drucksensors außerhalb des vorgegebenen Druckbereiches liegt,
v. Empfangen und Prüfen des Messwertes des Sauerstoffsensors während der Kompressionstherapie durch den Computer,
   wobei geprüft wird, ob der Messwert des Sauerstoffsensors eine vorgegebene Untergrenze für eine Sauerstoffsättigung unterschreitet, und
   wobei vorzugsweise ein Alarm ausgegeben wird, wenn die Prüfung ergibt, dass der Messwert des Sauerstoffsensors die vorgegebene Untergrenze für die Sauerstoffsättigung unterschreitet.

Das computerimplementierte Verfahren kann vorteilhaft mit sämtlichen der vorliegend beschriebenen Ausführungsformen des Kompressionstherapiesystems durchgeführt werden.

Die Erfindung umfasst ein Computerprogramm zur Durchführung des zuvor beschriebenen computerimplementierten Verfahrens. Beansprucht wird dabei ein

Computerprogrammprodukt, umfassend Befehle, die bei der Ausführung des Programmes durch ein erfindungsgemäßes Kompressionstherapiesystem dieses veranlassen, die Schritte i. bis v. (also alle Schritte) oder zumindest die Schritte iv. und v. des oben genannten computerimplementierten Verfahrens auszuführen. Beansprucht wird zudem ein computerlesbarer Datenträger, auf dem ein solches Computerprogrammprodukt gespeichert ist. Der computerlesbare Datenträger kann hierbei insbesondere eine CD, DVD oder ein USB-Stick sein. Schließlich wird noch ein Datenträgersignal beansprucht, das ein solches Computerprogrammprodukt überträgt. Das Datenträgersignal kann zum Beispiel von einer onlinebasierten Vertriebsplattform (App Store wie beispielsweise Google Play) ausgehen und das Computerprogramm auf ein Smartphone oder ein Tablet zur Installation übertragen.

### Beispiele und Figuren

Mit den nachfolgend beschriebenen Beispielen und Figuren soll die Erfindung exemplarisch näher erläutert und veranschaulicht werden.

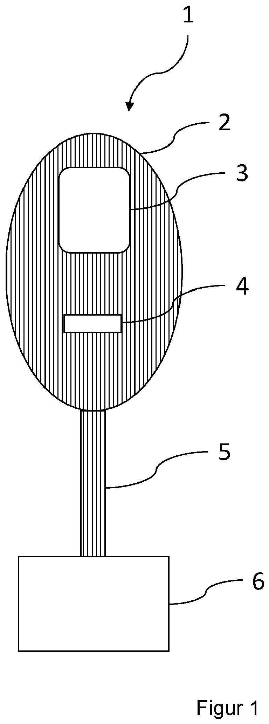

**Figur 1** zeigt eine Sensoreinheit **1.** Die Sensoreinheit **1** ist ein Bestandteil einer ersten bevorzugten Ausführungsform des erfindungsgemäßen Kompressionstherapiesystems. Die Sensoreinheit **1** umfasst ein Substrat **2,** bei dem es sich um eine bevorzugt flexible Leiterplatte handelt. Auf dem Substrat **2** ist ein Drucksensor **3,** zum Beispiel ein piezoelektrischer Drucksensor **3,** sowie ein reflexions-pulsoximetrischer Sauerstoffsensor **4** angeordnet. Typischerweise besitzt der Sauerstoffsensor zwei Lichtquellen, jeweils eine für rotes und eine für infrarotes Licht, sowie einen Photodetektor. Ein Konnektor **5** verbindet dann das Substrat **2** mit einer Datenerfassungseinheit **6.** Diese hat die Aufgabe, die Messwerte von dem Drucksensor **3** und von dem Sauerstoffsensor **4** zu erfassen und an einen Computer (nicht dargestellt) kabellos, beispielsweise mittels Bluetooth oder NFC, zu übertragen. Hierfür kann die Datenerfassungseinheit **6** einen Datenträger und einen Bluetooth- oder NFC-Sendeempfänger umfassen (nicht dargestellt). Als Computer ist insbesondere ein Smartphone vorgesehen. Der Computer kann die empfangenen Messdaten dann in der erfindungsgemäß vorgesehen Art und Weise prüfen und den Anwender alarmieren, wenn zum Beispiel der gemessene Druck zu hoch oder die gemessene Sauerstoffsättigung zu niedrig ist.

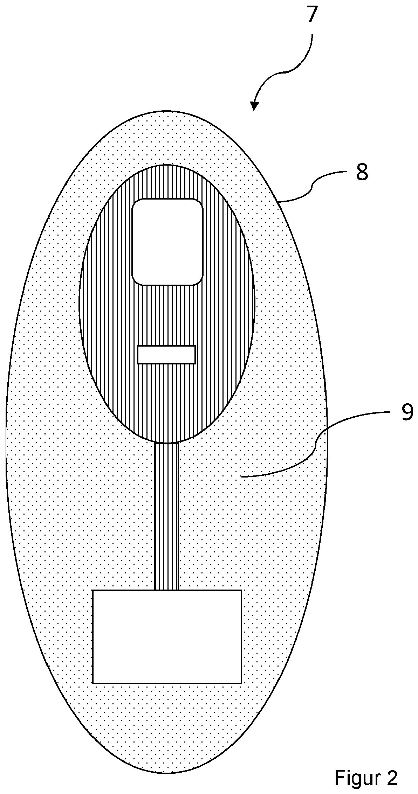

**Figur 2** zeigt eine Sensoreinheit **7** als Bestandteil einer zweiten bevorzugten Ausführungsform des erfindungsgemäßen Kompressionstherapiesystems. Die Sensoreinheit **7** unterscheidet sich von der Sensoreinheit **1** lediglich dadurch, dass sie in der Art eines Pflasters ausgebildet ist. Das pflasterartige Aussehen ergibt sich durch eine Rückschicht **8** ("backing"), welche auf einer Seite adhäsiv beziehungsweise klebend ausgerüstet ist und an dem Substrat **2,** dem Konnektor **5** und der Datenerfassungseinheit **6** zusätzlich befestigt ist. Mit dem freien, adhäsiven Randbereich **9** der Rückschicht **8** kann die Sensoreinheit am Körper des Patienten angeklebt werden. Die Rückschicht **8** aus **Figur 2** hat eine ovale Form. Die Rückschicht **8** kann aber auch eine andere Form, beispielsweise eine rechteckige Form, haben.

### Anwendungsbeispiel 1

- Zu behandelndes Körperteil: Unterschenkel eines Menschen
- Bestandteile des eingesetzten Kompressionstherapiesystems:
   - Sensoreinheit 1 oder Sensoreinheit 2 aus Figur 1 beziehungsweise Figur 2
   - eine Kompressionsbinde (als Kompressionsmittel, welches losgelöst von der Sensoreinheit bereitgestellt wird)
   - ein Smartphone (als Computer mit einem Datenträger, einem Bluetooth- und NFC-Sendeempfänger, einem Berührungsbildschirm sowie einem Lautsprecher)
- Zeitpunkt der Kontrollmessungen: erfolgt auf Befehl eines Benutzers hin
- Ablauf der Behandlung (in chronologischer Reihenfolge):
   i. **Befestigen der Sensoreinheit** an einer Stelle des Unterschenkels, an der Druck und Sauerstoffsättigung gemessen werden sollen. Für die Befestigung der Sensoreinheit 1 aus Figur 1 kann ein Heftpflaster aus dem medizinischen Bereich, zum Beispiel Omniplast^{®} von der Patentanmelderin, zu Hilfe genommen werden.
   ii. **Umwickeln des Unterschenkels mit der Kompressionsbinde.** Die Sensoreinheit ist dann zwischen Haut und Binde angeordnet. Der Zeitpunkt nach Anlegen der Kompressionsbinde kann als Beginn der Kompressionstherapie angesehen werden.
   iii. **Aktivieren einer auf dem Smartphone installierten Anwendung ("App"),** welche für die Verarbeitung der Sensormesswerte vorgesehen ist. In der Anwendung sind Referenzbereiche und Grenzwerte für Druck, Sauerstoffsättigung und Pulsschlag voreingestellt. Zum Beispiel könnte ein Druckbereich von 20 mmHg bis 40 mmHg, eine Untergrenze für die Sauerstoffsättigung von 85 % und ein Pulsbereich von 50 Schläge/min bis 150 Schläge/min voreingestellt sein. Falls erforderlich kann der Benutzer diese Voreinstellungen entsprechend den spezifischen medizinischen Gegebenheiten individuell anpassen.
   iv. **Starten einer Kontrollmessung** mit Hilfe der Anwendung. Messdaten von dem Drucksensor und dem Sauerstoffsensor werden dabei von der Sensoreinheit an das Smartphone übertragen. Im Ergebnis wird dem Benutzer der Druck angezeigt, welcher von der Kompressionsbinde auf den Unterschenkel ausgeübt wird. Zudem wird dem Benutzer die arterielle Sauerstoffsättigung am Unterschenkel sowie der Pulsschlag angezeigt. Falls einer dieser Parameter nicht mit den eingestellten Bereichen und Grenzwerten übereinstimmt, wird ein entsprechendes Alarmsignal ausgegeben. Damit wird sichergestellt, dass der Benutzer die Abweichung von der Norm in jedem Fall zur Kenntnis nimmt und als mögliche Gefahrensituation wahrnimmt.
   v. **Falls ein Alarmsignal ausgegeben wird, kann eine entsprechende Maßnahme zur Beseitigung des Alarmzustandes erfolgen.** Im Falle eines zu hohen Drucks oder einer zu niedrigen Sauerstoffsättigung kann zum Beispiel die Kompressionsbinde abgenommen und mit geringerem Zug wieder neu angelegt werden. Falls kein Alarmsignal ausgegeben wird, kann die Kompressionstherapie fortgesetzt werden. Der Benutzer hat dann Gewissheit, dass die Kompressionstherapie ordnungsgemäß abläuft.
   vi. **Zu einem späteren, von dem Benutzer frei wählbaren Zeitpunkt kann eine erneute Kontrollmessung durchgeführt werden.** Diese Kontrollmessung muss wie die Kontrollmessung im obigen Schritt iv. vom Benutzer initiiert werden, denn die Datenübertragung und die Datenüberprüfung findet nur auf Abruf statt. Dadurch kann die Menge der übertragenen Messdaten jedoch verringert und in Verbindung mit einem entsprechenden Übertragungsstandard (zum Beispiel NFC) auf eine Batterie für die Sensoren verzichtet werden.

### Anwendungsbeispiel 2

- Zu behandelndes Körperteil: Unterschenkel eines Menschen
- Bestandteile des eingesetzten Kompressionstherapiesystems: Das System ist wie bei dem Anwendungsbeispiel 1 ausgeführt. Als zusätzlichen Bestandteil besitzt die Sensoreinheit eine Batterie für den Druck- und Sauerstoffsensor.
- Zeitpunkt der Kontrollmessungen: automatisch und kontinuierlich
- Ablauf der Behandlung (in chronologischer Reihenfolge):
   i. **Befestigen der Sensoreinheit** (wie bei Anwendungsbeispiel 1).
   ii. **Umwickeln des Unterschenkels mit der Kompressionsbinde** (wie bei Anwendungsbeispiel 1).
   iii. **Aktivieren einer auf dem Smartphone installierten Anwendung** (wie bei Anwendungsbeispiel 1).
   iv. **Messdaten von dem Drucksensor und dem Sauerstoffsensor werden von der Sensoreinheit automatisch und kontinuierlich an das Smartphone übertragen.** Im Ergebnis kann die Anwendung dem Benutzer jederzeit den aktuellen, von der Kompressionsbinde auf den Unterschenkel ausgeübten Druck, die aktuelle arterielle Sauerstoffsättigung am Unterschenkel sowie den aktuellen Pulsschlag anzeigen. Falls einer dieser Parameter nicht mit den eingestellten Bereichen und Grenzwerten übereinstimmt, wird wie im vorangegangenen Anwendungsbeispiel ein entsprechendes Alarmsignal ausgegeben.
   v. **Sobald ein Alarmsignal ausgegeben wird, kann eine entsprechende Maßnahme zur Beseitigung des Alarmzustandes erfolgen** und danach die Kompressionstherapie fortgesetzt werden (wie bei Anwendungsbeispiel 1).
   vi. **Falls es bei der Fortsetzung der Kompressionstherapie erneut zu einem Alarmzustand kommt,** wird dieser durch die automatische und kontinuierliche Datenübertragung und Datenauswertung von der bereits aktiven Anwendung **selbstständig erkannt und an den Benutzer kommuniziert.** Dieses kontinuierliche Monitoring mit automatischer Alarmierung macht die Kompressionstherapie sehr sicher.

### Anwendungsbeispiel 3

- Zu behandelndes Körperteil: Unterschenkel eines Menschen
- Bestandteile des eingesetzten Kompressionstherapiesystems: Das System ist wie bei dem Anwendungsbeispiel 1 ausgeführt.
- Zeitpunkt der Referenz- und Kontrollmessungen: erfolgt auf Befehl eines Benutzers hin
- Ablauf der Behandlung (in chronologischer Reihenfolge):
   i. **Befestigen der Sensoreinheit an einer Stelle des Unterschenkels** (wie bei Anwendungsbeispiel 1).
   ii. **Aktivieren einer auf dem Smartphone installierten Anwendung** (wie bei Anwendungsbeispiel 1).
   iii. **Starten einer Referenzmessung** mit Hilfe der Anwendung. Messdaten von dem Sauerstoffsensor werden dabei von der Sensoreinheit an das Smartphone übertragen. Die Messung dient dem Zweck, den Grenzwert für die Sauerstoffsättigung festzulegen.
   iv. **Umwickeln des Unterschenkels mit der Kompressionsbinde** (wie bei Anwendungsbeispiel 1).
   v. **Starten einer Kontrollmessung** mit Hilfe der Anwendung (wie bei Anwendungsbeispiel 1 mit dem Unterschied, dass die Untergrenze für die Sauerstoffsättigung kein voreingestellter Wert, sondern die in Schritt iii. bestimmte Sauerstoffsättigung vor Beginn der Kompressionstherapie ist).
   vi. **Falls ein Alarmsignal ausgegeben wird, kann eine entsprechende Maßnahme zur Beseitigung des Alarmzustandes erfolgen** und danach die Kompressionstherapie fortgesetzt werden (wie bei Anwendungsbeispiel 1).
   vii. **Zu einem späteren, von dem Benutzer frei wählbaren Zeitpunkt kann eine erneute Kontrollmessung durchgeführt werden** (wie bei Anwendungsbeispiel 1).

## Patentansprüche

1. System für eine Kompressionstherapie, umfassend
- ein Kompressionsmittel,
- einen Drucksensor (3),
- einen Computer,
**dadurch gekennzeichnet, dass**
- das System einen pulsoximetrischen Sauerstoffsensor (4) umfasst, wobei Messwerte des Drucksensors (3) und des Sauerstoffsensors (4) an den Computer übertragen werden können, und
- der Computer Mittel zur Ausführung eines Verfahrens zur Überprüfung der Kompressionstherapie umfasst, wobei das Verfahren die folgenden Schritte umfasst:
i. Empfangen und Prüfen eines Messwertes des Drucksensors (3) während der Kompressionstherapie,
wobei geprüft wird, ob der Messwert des Drucksensors (3) außerhalb eines vorgegebenen Druckbereiches liegt, und
wobei vorzugsweise ein Alarm ausgegeben wird, wenn die Prüfung ergibt, dass der Messwert des Drucksensors (3) außerhalb des vorgegebenen Druckbereiches liegt,
ii. Empfangen und Prüfen eines Messwertes des Sauerstoffsensors (4) während der Kompressionstherapie,
wobei geprüft wird, ob der Messwert des Sauerstoffsensors (4) eine vorgegebene Untergrenze für eine Sauerstoffsättigung unterschreitet, und
wobei vorzugsweise ein Alarm ausgegeben wird, wenn die Prüfung ergibt, dass der Messwert des Sauerstoffsensors (4) die vorgegebene Untergrenze für die Sauerstoffsättigung unterschreitet.

2. System nach Anspruch 1, **dadurch gekennzeichnet, dass** das Kompressionsmittel eine Kompressionsbinde, ein Kompressionsschlauch oder ein Kompressionsstrumpf ist.

3. System nach Anspruch 1 oder 2, **dadurch gekennzeichnet, dass** es sich bei dem Drucksensor (3) um einen Dehnungsmessstreifen, einen induktiven Drucksensor oder einen piezoelektrischen Drucksensor handelt.

4. System nach einem der vorangehenden Ansprüche, **dadurch gekennzeichnet, dass** der Sauerstoffsensor (4) eine Lichtquelle für rotes Licht, eine Lichtquelle für infrarotes Licht und einen Photodetektor umfasst, wobei die Lichtquellen vorzugsweise Leuchtdioden sind und der Photodetektor vorzugsweise eine Photodiode ist.

5. System nach einem der vorangehenden Ansprüche, **dadurch gekennzeichnet, dass** es sich bei dem Sauerstoffsensor (4) um einen reflexions-pulsoximetrischen Sauerstoffsensor handelt.

6. System nach einem der vorangehenden Ansprüche, **dadurch gekennzeichnet, dass** es sich bei dem Computer um ein mobiles Gerät, insbesondere ein Smartphone oder ein Tablet, handelt.

7. System nach einem der vorangehenden Ansprüche, **dadurch gekennzeichnet, dass** die Übertragung der Messwerte des Drucksensors (3) und des Sauerstoffsensors (4) an den Computer kabellos erfolgt, wobei die Übertragung vorzugsweise über eine Nahfeldkommunikation, Bluetooth oder ein drahtloses lokales Netzwerk erfolgt.

8. System nach einem der vorangehenden Ansprüche, **dadurch gekennzeichnet, dass** das Empfangen und Prüfen der Messwerte des Drucksensors (3) und des Sauerstoffsensors (4) unmittelbar nachdem die Messwerte von den Sensoren erfasst wurden erfolgt.

9. System nach einem der vorangehenden Ansprüche, **dadurch gekennzeichnet, dass** das Empfangen und Prüfen des Messwertes des Drucksensors (3) und das Empfangen und Prüfen des Messwertes des Sauerstoffsensors (4) gleichzeitig erfolgen.

10. System nach einem der vorangehenden Ansprüche, **dadurch gekennzeichnet, dass** das Empfangen und Prüfen der Messwerte des Drucksensors (3) und des Sauerstoffsensors (4) automatisch und kontinuierlich oder zumindest automatisch in regelmäßigen Abständen nach Beginn der Kompressionstherapie erfolgt.

11. System nach einem der Ansprüche 1 bis 9, **dadurch gekennzeichnet, dass** das Empfangen und Prüfen der Messwerte des Drucksensors (3) und des Sauerstoffsensors (4) ausschließlich auf Befehl eines Benutzers hin erfolgt.

12. System nach einem der vorangehenden Ansprüche, **dadurch gekennzeichnet, dass**
- eine Untergrenze des Druckbereiches einen Wert von 1 mmHg bis 20 mmHg, bevorzugt 10 mmHg bis 20 mmHg und besonders bevorzugt 15 mmHg bis 20 mmHg aufweist, und
- eine Obergrenze des Druckbereiches einen Wert von 40 mmHg bis 200 mmHg, bevorzugt 40 mmHg bis 120 mmHg, mehr bevorzugt 40 mmHg bis 100 mmHg, noch mehr bevorzugt 40 mmHg bis 80 mmHg und besonders bevorzugt 40 mmHg bis 60 mmHg aufweist,
wobei die Untergrenze insbesondere einen Wert von 20 mmHg aufweist und die Obergrenze insbesondere einen Wert von 40 mmHg aufweist.

13. System nach einem der vorangehenden Ansprüche, **dadurch gekennzeichnet, dass** die Untergrenze für die Sauerstoffsättigung aus einem Bereich von 95 % bis 70 %, vorzugsweise 90 % bis 70 %, ausgewählt ist, wobei die Untergrenze für die Sauerstoffsättigung insbesondere 90 % oder 85 % beträgt.

14. System nach einem der Ansprüche 1 bis 12, **dadurch gekennzeichnet, dass** die Untergrenze für die Sauerstoffsättigung eine vor Beginn oder zu Beginn der Kompressionstherapie von dem Sauerstoffsensor (4) gemessene Sauerstoffsättigung ist.

15. System nach einem der vorangehenden Ansprüche, **dadurch gekennzeichnet, dass** der Computer Mittel zur Ausführung eines Verfahrens zur Überprüfung eines Pulsschlages umfasst, wobei das Verfahren den folgenden Schritt umfasst:
- Empfangen und Prüfen eines Messwertes des Sauerstoffsensors (4) während der Kompressionstherapie,
wobei geprüft wird, ob der Messwert des Sauerstoffsensors (4) außerhalb eines vorgegebenen Pulsbereiches liegt, und
wobei vorzugsweise ein Alarm ausgegeben wird, wenn die Prüfung ergibt, dass der Messwert des Sauerstoffsensors (4) außerhalb des vorgegebenen Pulsbereiches liegt.

16. System nach Anspruch 15, **dadurch gekennzeichnet, dass**
- eine Untergrenze des Pulsbereiches einen Wert von 40 Schläge/min bis 60 Schläge/min, insbesondere 50 Schläge/min, aufweist, und
- eine Obergrenze des Pulsbereiches einen Wert von 100 Schläge/min bis 200 Schläge/min, insbesondere 150 Schläge/min, aufweist.

17. Computerprogrammprodukt, umfassend Befehle, die bei der Ausführung des Programmes durch ein System nach einem der Ansprüche 1 bis 16 dieses veranlassen, die nachfolgenden Schritte i. bis v. oder zumindest die nachfolgenden Schritte iv. und v. eines computerimplementiertes Verfahren zur Überprüfung einer Kompressionstherapie auszuführen:
i. Erzeugen eines Messwertes mit einem Drucksensor (3), um einen von einem Kompressionsmittel auf eine Körperstelle ausgeübten Druck während der Kompressionstherapie zu bestimmen,
ii. Erzeugen eines Messwertes mit einem pulsoximetrischen Sauerstoffsensor (4), um eine Sauerstoffsättigung der mit dem Kompressionsmittel behandelten Körperstelle während der Kompressionstherapie zu bestimmen,
iii. Übertragen der Messwerte des Drucksensors (3) und des Sauerstoffsensors (4) an einen Computer,
iv. Empfangen und Prüfen des Messwertes des Drucksensors (3) während der Kompressionstherapie durch den Computer,
wobei geprüft wird, ob der Messwert des Drucksensors (3) außerhalb eines vorgegebenen Druckbereiches liegt, und
wobei vorzugsweise ein Alarm ausgegeben wird, wenn die Prüfung ergibt, dass der Messwert des Drucksensors (3) außerhalb des vorgegebenen Druckbereiches liegt,
v. Empfangen und Prüfen des Messwertes des Sauerstoffsensors (4) während der Kompressionstherapie durch den Computer,
wobei geprüft wird, ob der Messwert des Sauerstoffsensors (4) eine vorgegebene Untergrenze für eine Sauerstoffsättigung unterschreitet, und
wobei vorzugsweise ein Alarm ausgegeben wird, wenn die Prüfung ergibt, dass der Messwert des Sauerstoffsensors (4) die vorgegebene Untergrenze für die Sauerstoffsättigung unterschreitet.

18. Computerlesbarer Datenträger, auf dem das Computerprogrammprodukt nach Anspruch 17 gespeichert ist.

19. Datenträgersignal, das das Computerprogrammprodukt nach Anspruch 17 überträgt.

## Claims

1. System for a compression therapy, comprising
- a compression means,
- a pressure sensor (3),
- a computer,
**characterized in that**
- the system comprises a pulse oximeter oxygen sensor (4), wherein measurement values from the pressure sensor (3) and the oxygen sensor (4) can be transferred to the computer, and
- the computer comprises means for carrying out a method for monitoring the compression therapy, wherein the method comprises the following steps:
i. receiving and checking a measurement value from the pressure sensor (3) during the compression therapy,
wherein a check is carried out as to whether the measurement value from the pressure sensor (3) lies outside of a specified pressure range, and
wherein preferably an alarm is output if the check yields that the measurement value from the pressure sensor (3) lies outside of the specified pressure range,
ii. receiving and checking a measurement value from the oxygen sensor (4) during the compression therapy,
wherein a check is carried out as to whether the measurement value from the oxygen sensor (4) has dropped below a specified lower limit for an oxygen saturation, and
wherein preferably an alarm is output if the check yields that the measurement value from the oxygen sensor (4) has dropped below the specified lower limit for oxygen saturation.

2. System according to Claim 1, **characterized in that** the compression means is a compression bandage, a compression tube or a compression stocking.

3. System according to Claim 1 or 2, **characterized in that** the pressure sensor (3) is a strain gauge, an inductive pressure sensor or a piezoelectric pressure sensor.

4. System according to any of the preceding claims, **characterized in that** the oxygen sensor (4) comprises a light source for red light, a light source for infrared light and a photodetector, wherein the light sources are preferably light-emitting diodes, and the photodetector is preferably a photodiode.

5. System according to any of the preceding claims, **characterized in that** the oxygen sensor (4) is a reflection-type pulse oximeter oxygen sensor.

6. System according to any of the preceding claims, **characterized in that** the computer is a mobile device, in particular a smartphone or a tablet.

7. System according to any of the preceding claims, **characterized in that** the transfer of the measurement values from the pressure sensor (3) and from the oxygen sensor (4) to the computer is conducted wirelessly, wherein the transfer is preferably implemented by way of near field communication, Bluetooth or a wireless local network.

8. System according to any of the preceding claims, **characterized in that** the measurement values from the pressure sensor (3) and the measurement values from the oxygen sensor (4) are received and checked directly after the acquisition of the measurement values by the sensors.

9. System according to any of the preceding claims, **characterized in that** the measurement values from the pressure sensor (3) and the measurement values from the oxygen sensor (4) are received and checked simultaneously.

10. System according to any of the preceding claims, **characterized in that** the measurement values from the pressure sensor (3) and the measurement values from the oxygen sensor (4) are received and checked automatically and continuously or at least automatically at regular intervals following the start of the compression therapy.

11. System according to any of Claims 1 to 9, **characterized in that** the measurement values from the pressure sensor (3) and the measurement values from the oxygen sensor (4) are only received and checked following a command from the user.

12. System according to any of the preceding claims, **characterized in that**
- a lower limit of the pressure range has a value from 1 mmHg to 20 mmHg, preferably 10 mmHg to 20 mmHg and particularly preferably 15 mmHg to 20 mmHg, and
- an upper limit of the pressure range has a value from 40 mmHg to 200 mmHg, preferably 40 mmHg to 120 mmHg, more preferably 40 mmHg to 100 mmHg, even more preferably 40 mmHg to 80 mmHg and particularly preferably 40 mmHg to 60 mmHg,
wherein the lower limit in particular has a value of 20 mmHg and the upper limit in particular has a value 40 mmHg.

13. System according to any of the preceding claims, **characterized in that** the lower limit for the oxygen saturation is selected from a range of 95% to 70%, preferably 90% to 70%, wherein the lower limit for the oxygen saturation is in particular 90% to 85%.

14. System according to any of Claims 1 to 12, **characterized in that** the lower limit for the oxygen saturation is an oxygen saturation measured by the oxygen sensor (4) prior to the start or at the start of the compression therapy.

15. System according to any of the preceding claims, **characterized in that** the computer comprises means for carrying out a method for monitoring a pulsebeat, wherein the method comprises the following step:
- receiving and checking a measurement value from the oxygen sensor (4) during the compression therapy, wherein a check is carried out as to whether the measurement value from the oxygen sensor (4) lies outside of a specified pulse range and
wherein preferably an alarm is output if the check yields that the measurement value from the oxygen sensor (4) lies outside of the specified pulse range.

16. System according to Claim 15, **characterized in that**
- a lower limit of the pulse range has a value of 40 beats/min to 60 beats/min, in particular 50 beats/min and
- an upper limit of the pulse range has a value of 100 beats/min to 200 beats/min, in particular 150 beats/min.

17. Computer program product comprising commands which, when the program is executed by a system according to any of Claims 1 to 16, cause the said system to carry out steps i. to v., below, or at least steps iv. and v., below, of a computer-implemented method for monitoring a compression therapy:
i. generating a measurement value using a pressure sensor (3) in order to determine a pressure exerted by a compression means on a body location during the compression therapy,
ii. generating a measurement value using a pulse oximeter oxygen sensor (4) in order to determine an oxygen saturation of the body location, treated by the compression means, during the compression therapy,
iii. transferring the measurement values from the pressure sensor (3) and from the oxygen sensor (4) to a computer,
iv. receiving and checking the measurement values from the pressure sensor (3) by the computer during the compression therapy,
wherein a check is carried out as to whether the measurement value from the pressure sensor (3) lies outside of a specified pressure range, and
wherein preferably an alarm is output if the check yields that the measurement value from the pressure sensor (3) lies outside of the specified pressure range,
v. receiving and checking the measurement value from the oxygen sensor (4) by the computer during the compression therapy,
wherein a check is carried out as to whether the measurement value from the oxygen sensor (4) has dropped below a specified lower limit for an oxygen saturation, and
wherein preferably an alarm is output if the check yields that the measurement value from the oxygen sensor (4) has dropped below the specified lower limit for the oxygen saturation.

18. Computer-readable data carrier, on which the computer program product according to Claim 17 is stored.

19. Data carrier signal which transfers the computer program product according to Claim 17.

## Revendications

1. Système, pour une thérapie par compression, comprenant
- un moyen de compression,
- un capteur de pression (3),
- un ordinateur,
**caractérisé en ce que**
- le système comprend un capteur d'oxygène (4) par pulsoxymétrie, des valeurs mesurées par le capteur de pression (3) et par le capteur d'oxygène (4) pouvant être transférées à l'ordinateur, et
- l'ordinateur comprend des moyens destinés à vérifier la thérapie par compression, le procédé comprenant les étapes suivantes, consistant à :
i. réceptionner et vérifier une valeur mesurée par le capteur de pression (3) au cours de la thérapie par compression,
étant vérifié si la valeur mesurée par le capteur de pression (3) se situe à l'extérieur d'une plage de pression prédéfinie, et
de préférence une alarme étant délivrée s'il résulte de la vérification que la valeur mesurée par le capteur de pression (3) se situe à l'extérieur de la plage de pression prédéfinie,
ii. réceptionner et vérifier une valeur mesurée par le capteur d'oxygène (4) au cours de la thérapie par compression,
étant vérifié si la valeur mesurée par le capteur d'oxygène (4) est inférieure à une limite inférieure prédéfinie pour une saturation d'oxygène, et une alarme étant délivrée de préférence s'il résulte de la vérification que la valeur mesurée par le capteur d'oxygène (4) est inférieure à la limite inférieure pour la saturation d'oxygène.

2. Système selon la revendication 1, **caractérisé en ce que** le moyen de compression est une bande de compression, un manchon de compression ou un bas de compression.

3. Système selon la revendication 1 ou 2, **caractérisé en ce que** le capteur de pression (3) est une jauge de contrainte, un capteur de pression inductif ou un capteur de pression piézoélectrique.

4. Système selon l'une quelconque des revendications précédentes, **caractérisé en ce que** le capteur d'oxygène (4) comprend une source lumineuse pour lumière rouge, une source lumineuse pour une lumière infrarouge et un photocapteur, les sources lumineuses étant de préférence des diodes électroluminescentes et le photocapteur étant de préférence une photodiode.

5. Système selon l'une quelconque des revendications précédentes, **caractérisé en ce que** le capteur d'oxygène (4) est un capteur d'oxygène par pulsoxymétrie par réflexion.

6. Système selon l'une quelconque des revendications précédentes, **caractérisé en ce que** l'ordinateur est un appareil mobile, notamment un smartphone ou une tablette.

7. Système selon l'une quelconque des revendications précédentes, **caractérisé en ce que** la transmission des valeurs mesurées par le capteur de pression (3) et par le capteur d'oxygène (4) à l'ordinateur s'effectue sans fil, la transmission s'effectuant de préférence via une communication en champ proche, via Bluetooth ou via un réseau local sans fil.

8. Système selon l'une quelconque des revendications précédentes, **caractérisé en ce que** la réception et la vérification des valeurs mesurées par le capteur de pression (3) et par le capteur d'oxygène (4) s'effectue directement dès que les valeurs mesurées ont été détectées par les capteurs.

9. Système selon l'une quelconque des revendications précédentes, **caractérisé en ce que** la réception et la vérification de la valeur mesurée par le capteur de pression (3) et la réception et la vérification de la valeur mesurée par le capteur d'oxygène (4) s'effectuent simultanément.

10. Système selon l'une quelconque des revendications précédentes, **caractérisé en ce que** la réception et la vérification de la valeur mesurée par le capteur de pression (3) et par le capteur d'oxygène (4) s'effectue automatiquement en en continu ou au moins automatiquement à intervalles réguliers après le début de la thérapie par compression.

11. Système selon l'une quelconque des revendications 1 à 9, **caractérisé en ce que** la réception et la vérification de la valeur mesurée par le capteur de pression (3) et par le capteur d'oxygène (4) s'effectue exclusivement en réponse à une commande donnée par un utilisateur.

12. Système selon l'une quelconque des revendications précédentes, **caractérisé en ce**
- **qu'**une limite inférieure de la plage de pression présente une valeur de 1 mmHg à 20 mmHg, de préférence de 10 mmHg à 20 mmHg et de manière particulièrement préférentielle, de 15 mmHg à 20 mmHg,
- en ce qu'une limite supérieure de la plage de pression présente une valeur de 40 mmHg à 200 mmHg, de préférence de 40 mmHg à 120 mmHg, de manière plus préférentielle, de 40 mmHg à 100 mmHg, de manière encore plus préférentielle, de 40 mmHg à 80 mmHg et de manière particulièrement préférentielle, de 40 mmHg à 60 mmHg,
la limite inférieure présentant notamment une valeur de 20 mmHg et la limite supérieure présentant notamment une valeur de 40 mmHg.

13. Système selon l'une quelconque des revendications précédentes, **caractérisé en ce que** la limite inférieure pour la saturation d'oxygène est sélectionnée dans une plage de 95 % à 70 %, de préférence de 90 % à 70 %, la limite inférieure pour la saturation d'oxygène s'élevant notamment à 90 % ou à 85 %.

14. Système selon l'une quelconque des revendications 1 à 12, **caractérisé en ce que** la limite inférieure pour la saturation d'oxygène est une saturation d'oxygène mesurée par le capteur d'oxygène (4) avant le début ou au début de la thérapie par compression.

15. Système selon l'une quelconque des revendications précédentes, **caractérisé en ce que** l'ordinateur comprend des moyens destinés à réaliser un procédé de vérification d'un pouls, le procédé comprenant les étapes suivantes, consistant à :
- réceptionner et vérifier une valeur mesurée par le capteur d'oxygène (4) au cours de la thérapie par compression,
étant vérifié si la valeur mesurée par le capteur d'oxygène (4) se situe à l'extérieur d'une plage de pouls prédéfinie, et
de préférence une alarme étant délivrée, s'il résulte de la vérification que la valeur mesurée par le capteur d'oxygène (4) se situe à l'extérieur de la plage de pouls prédéfinie.

16. Système selon la revendication 15, **caractérisé en ce**
- **qu'**une limite inférieure de la plage de pouls présente une valeur de 40 pulsations/mn à 60 pulsations/mn, notamment de 50 pulsations/mn, et
- en ce qu'une limite supérieure de la plage de pouls présente une valeur de 100 pulsations/mn à 200 pulsations/mn, notamment de 150 pulsations/mn.

17. Produit de programme informatique, comprenant des commandes qui lors de l'exécution du programme par un système selon l'une quelconque des revendications 1 à 16 incitent ce dernier à réaliser les étapes i. à v. suivantes ou au moins les étapes iv. et v. suivantes d'un procédé mis en œuvre par ordinateur, destiné à vérifier une thérapie par compression et consistant à :
i. créer une valeur mesurée à l'aide du capteur de pression (3), pour déterminer une pression exercée sur une zone corporelle par un moyen de compression au cours de la thérapie par compression,
ii. créer une valeur mesurée à l'aide d'un capteur d'oxygène (4) de pulsoxymétrie, pour déterminer une saturation d'oxygène de la zone corporelle traitée par le moyen de compression au cours de la thérapie par compression,
iii. transmettre les valeurs mesurées par le capteur de pression (3) et par le capteur d'oxygène (4) à un ordinateur,
iv. réceptionner et vérifier via l'ordinateur la valeur mesurée par le capteur de pression (3) au cours de la thérapie par compression,
étant vérifié si la valeur mesurée par le capteur de pression (3) se situe à l'extérieur d'une plage de pression prédéfinie, et
de préférence, une alarme étant délivrée s'il résulte de la vérification que la valeur mesurée par le capteur de pression (3) se situe à l'extérieur de la plage de pression prédéfinie,
v. réceptionner et vérifier via l'ordinateur la valeur mesurée par le capteur d'oxygène (4) au cours de la thérapie par compression,
étant vérifié si la valeur mesurée par le capteur d'oxygène (4) est inférieure à une limite inférieure prédéfinie pour une saturation d'oxygène, et
de préférence une alarme étant délivrée s'il résulte de la vérification que la valeur mesurée par le capteur d'oxygène (4) est inférieure à la limite inférieure pour la saturation d'oxygène.

18. Support de données lisible par ordinateur, sur lequel est mémorisé le produit de programme informatique selon la revendication 17.

19. Signal porteur de données, qui transmet le produite de programme informatique selon la revendication 17.
